(19) Europäisches Patentamt
European Patent Office
Office européen des brevets

(11) Veröffentlichungsnummer: **0 269 078 B1**

(12) **EUROPÄISCHE PATENTSCHRIFT**

(45) Veröffentlichungstag der Patentschrift: **15.04.92**

(51) Int. Cl.$^5$: **C07C 69/533**, C07C 67/333

(21) Anmeldenummer: **87117355.5**

(22) Anmeldetag: **25.11.87**

(54) **Verfahren zur Isomerisierung von 2-cis-Pentensäureestern zu 2-trans-Pentensäureestern.**

(30) Priorität: **27.11.86 DE 3640598**

(43) Veröffentlichungstag der Anmeldung:
**01.06.88 Patentblatt 88/22**

(45) Bekanntmachung des Hinweises auf die Patenterteilung:
**15.04.92 Patentblatt 92/16**

(84) Benannte Vertragsstaaten:
**BE CH DE ES FR GB IT LI NL**

(56) Entgegenhaltungen:
**EP-A- 269 043**
**DE-A- 2 517 377**

(73) Patentinhaber: **BASF Aktiengesellschaft**
**Carl-Bosch-Strasse 38**
**W-6700 Ludwigshafen(DE)**

(72) Erfinder: **Fischer, Rolf, Dr.**
**Bergstrasse 98**
**W-6900 Heidelberg(DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Rank Xerox (UK) Business Services

## Beschreibung

Bei der Isomerisierung von 3-Pentensäureestern zu 4-Pentensäureestern in Gegenwart von Palladium, Rhodium oder Ruthenium enthaltenden Katalysatoren treten als Nebenprodukte 2-cis- und 2-trans-Pentensäureester auf, wie aus der DE-OS 33 17 163 zu entnehmen ist. 2-cis-Pentensäuremethylester besitzt den gleichen Siedepunkt wie 4-Pentensäuremethylester und läßt sich daher durch Destillation nicht von 4-Pentensäuremethylester abtrennen.

Es war daher die technische Aufgabe gestellt, 2-cis-Pentensäurester in Isomere umzuwandeln, die sich aufgrund ihres Siedepunktes durch Destillation von 4-Pentensäureestern abtrennen lassen, wobei gleichzeitig der gegebenenfalls vorhandene 4-Pentensäureester keiner Umwandlung unterliegt.

Diese Aufgabe wird gelöst in einem Verfahren zur Isomerisierung von 2-cis-Pentensäureestern zu 2-trans-Pentensäureestern, wobei man 2-cis-Pentensäureester in Gegenwart von mindestens einer Verbindung der Formel I

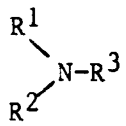 I,

in der $R^1$ ein Wasserstoffatom oder wie $R^2$ einen Alkylrest mit 1 bis 6 Kohlenstoffatomen oder einen Cycloalkylrest mit 5 bis 8 Kohlenstoffatomen bezeichnet, wobei $R^1$ und $R^2$ gemeinsam mit dem Stickstoffatom, das sie substituieren einen 5- bis 7-gliedrigen Ring bilden können, der zusätzlich ein weiteres Stickstoff- oder Sauerstoffatom enthalten kann und $R^3$ einen Wasserstoffatom oder einen Rest der Formel II

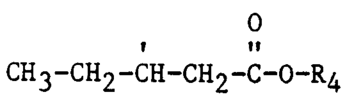 II

bezeichnet, in dem $R_4$ einen Alkylrest mit 1 bis 6 Kohlenstoffatomen, einen Cycloalkylrest mit 5 bis 7 Kohlenstoffatomen, einen Aralkylrest mit 7 bis 10 Kohlenstoffatomen oder einen Arylrest mit 6 bis 10 Kohlenstoffatomen bedeutet mit der Maßgabe, daß ein tert. Amin nur dann vorliegt, wenn $R^3$ einen Rest der Forme II bezeichnet, bei einer Temperatur von 20 bis 250 °C behandelt und daß man pro Mol 2-cis Pentensäureester 0,05 bis 0,2 Mol Verbindungen der Formel I verwendet.

Das neue Verfahren hat den Vorteil, daß es auf einfache Weise gelingt, 2-cis-Pentensäureester in 2-trans-Pentensäureester umzuwandeln, die sich aufgrund ihres Siedepunktes leicht durch Destillation von den entsprechenden 4-Pentensäureestern abtrennen lassen. Das neue Verfahren hat auch den Vorteil, daß bei der Isomerisation vorhandene 4-Pentensäureester praktisch keine Umwandlung erfahren.

Bevorzugte 2-cis-Pentensäureester leiten sich von Alkanolen mit 1 bis 6 Kohlenstoffatomen, Cycloalkanolen mit 5 bis 7 Kohlenstoffatomen, Aralkanolen mit 7 bis 10 Kohlenstoffatomen oder Phenol oder Naphthol ab. Besondere Bedeutung haben die entsprechenden Alkylester erlangt, insbesondere von Alkanolen mit bis zu 3 Kohlenstoffatomen. Geeignete Ausgangsverbindungen sind beispielsweise 2-cis-Pentensäuremethylester, -ethylester, n-propylester, -i-propylester, -tert. butylester, -i-butylester, -n-butylester, -sec. butylester, -n-pentylester, -n-hexylester, -dodecylester -cyclopentylester, -cyclohexylester, -cycloheptylester, -benzylester, -phenylethylester oder -phenylester. Die 2-cis-Pentensäureester können auch im Gemisch mit den entsprechenden 4-, 3-cis- und 3-trans- und/oder 2-trans-Pentensäureestern vorliegen. Typische Gemische enthalten beispielsweise 0,1 bis 99,5 Gew.-%, 4-Pentensäurester, 0,1 bis 99,5 Gew.-%, 3-cis- und trans-Pentensäureester, 0,1 bis 99,5 Gew.-%. 2-trans-Pentensäureester sowie 0,1 bis 100 Gew.-% 2-cis-Penensäurester.

Die Isomerisierung wird in Gegenwart von mindestens einer Verbindung der Formel I durchgeführt, in der $R^1$ ein Wasserstoffatom oder wie $R^2$ einen Cykloalkylrest mit 5 bis 8 Kohlenstoffatomen oder einen Alkylrest mit 1 bis 6 Kohlenstoffatomen bezeichnet, wobei $R^1$ und $R^2$ gemeinsam mit dem Stickstoffatom, das sie substituieren, einen 5- bis 7-gliedrigen Ring bilden können, der zusätzlich ein weiteres Stickstoff- oder Sauerstoffatom enthalten kann und $R^3$ ein Waserstoffatom oder einen Rest der Formel II bezeichnet, in der $R^4$ einen Alkylrest mit 1 bis 6-Kohlenstoffatomen, einen Cycloalkylrest mit 5 bis 7 Kohlenstoffatomen einen Aralkylrest mit 7 bis 10 Kohlenstoffatomen oder einen Arylrest mit 6 bis 10 Kohlenstoffatomen bezeichnet mit der Maßgabe, daß ein tert. Amin nur dann vorliegt, wenn $R^3$ einen Rest der Formel II

bedeutet.

Geeignete Verbindungen sind beispielsweise primäre oder sekundäre Amine der Formel I wie Methylamin, Ethylamin, n-Butylamin, i-Butylamin, n-Hexylamin, Cyclohexylamin, Cyclopentylamin, di-n-Butylamin oder Dicyclohexylamin.

In bevorzugten Aminen der Formel I bezeichnet $R^3$ ein Wasserstoffatom und $R^1$ und $R^2$ bilden zusammen mit dem Stickstoffatom, das sie substituieren einen 5- bis 7-gliedrigen Ring, der zusätzlich ein Stickstoff oder Sauerstoffatom enthalten kann. Beispiele hierfür sind Pyrrolidin, Piperidin, Piperazin, Morpholin oder Hexamethylenimin.

Weitere bevorzugte Katalysatoren sind tert. Amine der Formel I, in der $R^1$ und $R^2$ einen 5- oder 6-gliedrigen Ring bilden, der noch ein Sauerstoffatom oder Stickstoffatom enthalten kann und $R^3$ einen Rest der Formel II bezeichnet, wobei $R^4$ insbesondere einen Alkylrest mit 1 bis 6 Kohlenstoffatomen bezeichnet. Geeignete Verbindungen sind beispielswiese 3-Piperidino-, 3-Pyrrolidino-, 3-Morpholino-, 3-Piperazinovaleriansäuremethylester, 3-Piperidinovaleriansäurepropylester, 3-Pyrrolidinovaleriansäurecyclohexylester, 3-Piperazinovaleriansäurebenzylester oder 3-Morpholinovaleriansäurephenylester.

Je Mol 2-cis-Pentensäureester wendet man, 0,05 bis 0,2 Mol Verbindungen der Formel I an.

Die Isomerisierung wird bei einer Temperatur von 20 bis 250°C, insbesondere 50 bis 200°C durchgeführt. In der Regel führt man die Umsetzung bei Atmosphärendruck durch. Es ist aber auch möglich, Unterdruck oder schwachen Überdruck, z.B. bis zu 10 bar anzuwenden. Die Isomerisierung wird im allgemeinen diskontinuierlich oder kontinuierlich, z.B. in einer homogenen Flüssigphase durchgeführt.

Obwohl man die Isomerisierung im allgemeinen ohne Mitverwendung von Lösungsmitteln durchführt, ist es auch möglich, unter Reaktionsbedingungen inerte organische Lösungsmittel mitzuverwenden, wie aliphatische oder cycloaliphatische Kohlenwasserstoffe, z.B. Pentane, Hexane, Cyclohexan, aromatische Kohlenwasserstoffe, z.B. Benzol, Toluol, Xylole, ferner Ether, z.B. Methyltertiärbutylether, Dioxan, Tetrahydrofuran oder Ester, wie Essigsäureethylester.

Die diskontinuierliche Isomerisierung von 2-cis- zu 2-trans-Pentensäureester wird beispielsweise so durchgeführt, daß man 2-cis-Pentensäureester gegebenenfalls im Gemisch mit 2-trans, 3- und/oder 4-Pentensäureester zusammen mit der vorgenannten Menge an Verbindungen der Formel I auf die angegebene Temperatur erhitzt. Nach einer Reaktionszeit, z.B. von 0,5 bis 5 Stunden wird der Katalysator 1 zweckmäßig durch Destillation vom Pentensäureester-Isomerengemissch getrennt. Die als Katalysator verwendete Verbindung I kann widerum in die Isomerisierungsstufe zurückgeführt werden. Das Pentensäureestergemisch wird dann in der Regel durch Destillation aufgearbeitet.

Das erfindungsgemäße Verfahren ermöglicht es, z.B. 2-cis-Pentensäuremethylester durch Umwandlung in 2-trans-Pentensäuremethylester in eine von 4-Pentensäuremethylester durch Destillation abtrennbare Form zu überführen.

Die nach dem erfindungsgemäßen Verfahren erhältlichen 2-trans-Pentensäureester lassen sich zu 3-Pentensäureestern isomerisieren, die wichtige Ausgangsprodukte für die Herstellung von Adipinsäure sind.

Beispiel 1

Isomerisierung von 2-cis-Pentensäuremethylester mit Piperidin in Gegenwart von 2-trans-, 3- und 4-Pentensäuremethylester

Ein Gemisch aus 2,3 g 2-cis, 2,3 g 2-trans-, 16 g 3-, 2,3 g 4-Pentensäuremethylester (GC: 10,0 Fl.-% 2-cis, 10,4 Fl.-% 2-trans, 67,9 Fl-% 3-, 10,2 Fl.-%, 4-Pentensäuremethylester) wurde mit 1,7 g Piperidin 4 Stunden auf 65°C erhitzt. Die gaschromatographische Analyse der Pentenester (Flächen-%) ergab nach dieser Zeit, daß im Reaktionsgemisch 1,0 % 2-cis-, 19,5 % 2-trans-, 66,5 % 3- und 10,1 % 4-Pentensäuremethylester enthalten waren.

Beispiel 2

Isomerisierung von 2-cis-Pentensäuremethylester mit Piperidin in Gegenwart von 4-Pentensäuremethylester

Ein Gemisch aus 2,3 g 2-cis- und 20,5 g 4-Pentensäuremethylester wurde zusammen mit 1,7 g Piperidin 4 Stunden lang auf 65°C erhitzt. Die gaschromatographische Analyse (Flächen-%) der Pentenester ergab nach dieser Zeit, daß im Reaktionsgemisch 0,8 % 2-cis-, 9,0 % 2-trans, 0,1 % 3-cis- + trans- und 89,8 % 4-Pentensäuremethylester enthalten waren.

Beispiel 3 bis 8

Wie in den Beispielen 1 und 2 beschrieben, wurden die Beispiele 3 bis 8 durchgeführt. Tab. 1 enthält die Einsatzstoffe und Mengen, Reaktionsbedingungen und gaschromatographischen Analysen. In allen Beispielen wurden 20 g Pentenester-Isomerengemisch eingesetzt.

Tabelle 1

| Beispiel Nr. | Eingesetztes PSE-Gemisch[1)] | Katalysator [Mol-% bez. 2-cis-PSE] | Reaktionstemperatur [°C] | Reaktionszeit [h] | GC [Fl.-%] 2-cis-PSE | 2-trans-PSE | 3-PSE | 4-PSE |
|---|---|---|---|---|---|---|---|---|
| 3 | 20 % 2-cis-, 80 % 4- | n-Hexylamin 10 | 130 | 4 | 8,5 | 11,2 | 0,3 | 78,5 |
| 4 | 20 % 2-cis-, 80 % 4- | Cyclohexylamin 10 | 190 | 3 | 4,4 | 14,0 | 1,9 | 79,3 |
| 5 | 30 % 2-cis-, 70 % 4- | Piperazin 1 | 180 | 4 | 7,6 | 21,5 | 1,3 | 69,7 |
| 6 | 20 % 2-cis-, 80 % 4- | Morpholin 5 | 160 | 4 | 7,7 | 12,2 | 0,3 | 79,3 |
| 7 | 20 % 2-cis-, 80 % 4- | Pyrrolidin 5 | 160 | 2 | 2,6 | 16,7 | 0,8 | 79,2 |
| 8 | 30 % 2-cis-, 70 % 4- | Di-n-Butylamin 10 | 140 | 4 | 20,7 | 6,9 | 2,5 | 69,8 |

1) PSE = Pentensäuremethylester

Beispiel 9

a) Herstellung von 3-Piperidinovaleriansäuremethylester

Ein Gemisch aus 500 g 2-trans-Pentensäuremethylester und 372 g Piperidin wurde 17 Stunden lang auf 65 ° C, dann 24 Stunden lang auf 100 ° C erhitzt. Nach Destillation an einer Vigreux-Kolonne wurden 562 g 3-Piperidinovaleriansäuremethylester vom Siedepunkt 64 ° C/2 mbar, $n_D^{20}$ = 1,4592 erhalten (64 % d.Th.). alpha = 0,65 - 1,90 (m, 11 H), 1,90 - 3,20 (m, 7 H), 3,65 ppm (s,

b) Isomerisierung von 2-cis-Pentensäuremethylester mit 3-Piperidinovaleriansäuremethylester in Gegenwart von 4-Pentensäuremethylester

Ein Gemisch aus 10 g 2-cis- (50 %), 10 g 4-Pentensäuremethylester (50 %) und 1,75 g 3-Piperidinovaleriansäuremethylester wurde 2 Stunden lang auf 160 ° C erhitzt. Die gaschromatographische Analyse (Flächen-%) zeigte, daß das Pentenester-Isomerengemisch nach dieser Zeit zu 4,7 % aus 2-cis-, zu 44,1 % aus 2-trans-, zu 1,2 % aus 3-cis- + trans- und 49,3 % aus 4-Pentensäuremethylester bestand.

Beispiel 10

a) Herstellung von 3-Morpholinovaleriansäuremethylester

Ein Gemisch aus 171 g 2-trans-Pentensäuremethylester und 130,5 g Morpholin wurde 46 Stunden lang auf 100 ° C erhitzt. Nach Destillation des Reaktionsgemisches über eine Vigreux-Kolonne wurden 179 g 3-Morpholinovaleriansäuremethylester vom Siedepunkt 85 ° C/0,4 mbar, $n_D^{20}$ = 1,4610 erhalten (59 % d.Th.).
alpha = 0,70 - 1,80 (m, 5 H), 2,0 - 3,2 (m, 7 H), 3,45 - 3,83 (m, 4H), 3,68 ppm (s. 3 H)

b) Isomerisierung von 2-cis-Pentensäuremethylester in Gegenwart von 4-Pentensäuremethylester

Ein Gemisch aus 14 g 2-cis-, 6 g 4-Pentensäuremethylester und 4,9 g 3-Morpholinovaleriansäuremethylester wurde in einem Glasautoklaven 3 Stunden lang auf 180 ° C erhitzt. Nach dieser Zeit ergab die gaschromatographische Analyse (Flächen-%), daß das Reaktionsgemisch zu 6,6 % aus 2-cis-, zu 59,9 % aus 2-trans- und zu 5,1 % aus 3-cis-und trans-Pentensäuremethylester bestand.

Beispiel 11

a) Herstellung von 3-Piperazinovaleriansäuremethylester

Ein Gemisch aus 213 g 2-trans-Pentensäuremethylester und 64 g Piperazin wurde 44 Stunden lang bei 60 ° C gerührt. Durch fraktionierende Destillation wurden 77,1 g 3-Piperazinovaleriansäuremethylester vom Siedepunkt 105 ° C/0,4 mbar, $n_D^{20}$ = 1,4740 erhalten (51 %, bezogen auf eingesetztes Piperazin) delta = 0,70 - 1,80 (m, 5 H), 1,72 (s, 1 H), 2,0 - 3,2 (m, 11 H), 3,70 ppm (s, 3 H).

b) Isomerisierung von 2-cis-Pentensäuremethylester

Ein Gemisch aus 15 g 2-cis-Pentensäuremethylester und 2,6 g 3-Piperazinovaleriansäuremethylester wurde 4 Stunden lang auf 130 ° C erhitzt. Die gaschromatographische Analyse ergab nach dieser Reaktionszeit, daß das Reaktionsgemisch zu 5 % aus 2-cis, zu 91 % aus 2-trans- und zu 4 % aus 3-cis- und trans-Pentensäuremethylester bestand.

c) Isomerisierung von 2-cis-Pentensäuremethylester in Gegenwart von 4-Pentensäuremethylester

Ein Gemisch aus 10 g 2-cis-, 10 g 4-Pentensäuremethylester und 1,75 g 3-Piperazinovaleriansäuremethylester wurde 4 Stunden lang auf 130 ° C erhitzt. Nach dieser Zeit zeigte die gaschromatographische Analyse, daß das Reaktionsgemisch zu 3,9 % aus 2-cis-, zu 45 % aus 2-trans-, zu 1,4 % aus 3-cis- + trans- und 49,5 ^ aus 4-Pentensäuremethylester bestand.

Beispiel 12

a) Herstellung von 3-Pyrrolidinovaleriansäuremethylester

Ein Gemisch aus 171 g 2-trans-Pentensäuremethylester und 106,5 g Pyrrolidin wurde 20 Stunden lang auf 100 ° C erhitzt. Nach Destillation über eine Vigreux-Kolonne wurden 198,7 g 3-Pyrrolidinovaleriansäuremethylester vom Siedepunkt 65 ° C/0,4 mbar, $n_C^{20}$ = 1,4580 erhalten (72 % d.Th.).
delta = 0,89 (t, 3 H), 1,20 - 2,0 (m, 6 H), 2,10 - 3,10 (m, 7 H), 3,65 ppm (s, 3 H)

b) Isomerisierung von 2-cis-Pentensäuremethylester

Ein Gemisch aus 15 g 2-cis-Pentensäuremethylester und 2,4 g 3-Pyrrolidinovaleriansäuremethylester wurde 0,5 Stunden lang in einem Glasautoklaven auf 180 ° C erhitzt. Nach dieser Zeit ergab die gaschromatographische Analyse, daß das Reaktionsgemisch zu 32 % aus 2-cis-, zu 65 % aus 2-trans- und zu 4 % aus 3-cis- + trans-Pentensäuremethylester bestand.

**Patentansprüche**

1. Verfahren zur Isomerisierung von 2-cis-Pentensäureestern zu 2-trans-Pentensäureestern, dadurch gekennzeichnet, daß man 2-cis-Pentensäureester in Gegenwart von mindestens einer Verbindung der Formel I

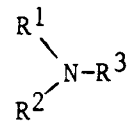

I,

in der $R^1$ ein Wasserstoffatom oder wie $R^2$ einen Cycloalkylrest mit 5 bis 8 Kohlenstoffatomen oder einen Alkylrest mit 1 bis 6 Kohlenstoffatomen bezeichnet, wobei $R^1$ und $R^2$ gemeinsam mit dem Stickstoffatom, das sie substituieren, einen 5- bis 7-gliedrigen Ring bilden können, der zusätzlich ein weiteres Stickstoff- oder Sauerstoffatom enthalten kann und $R^3$ ein Wasserstoffatom oder einen Rest der Formel II

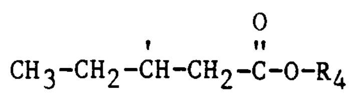

II

bezeichnet, in der $R_4$ einen Alkylrest mit 1 bis 6 Kohlenstoffatomen, einen Cycloalkylrest mit 5 bis 7 Kohlenstoffatomen, einen Aralkylrest mit 7 bis 10 Kohlenstoffatomen oder einen Arylrest mit 6 bis 10 Kohlenstoffatomen bezeichnet, mit der Maßgabe, daß ein tert. Amin nur dann vorliegt, wenn $R^3$ den Rest der Formel II bedeutet, bei einer Temperatur von 20 bis 250 °C behandelt und daß man pro Mol 2-cis-Pentensäureester 0,05 bis 0,2 Mol Verbindungen der Formel I verwendet.

**2.** Verfahren nach Anspruch 1, daduch gekennzeichnet, daß man als Verbindung der Formel I Piperidin, Pyrrolidin, Morpholin oder Piperazin verwendet.

**3.** Verfahren nach Anspruch 1, dadurch gekennzeichnet, daß man als Verbindung der Formel I 3-Piperidino-, 3-Pyrrolidino-, 3-Morpholino-oder 3-Piperazinovaleriansäureester verwendet.

## Claims

**1.** A process for the isomerization of 2-cis-pentenoates to 2-trans-pentenoates, wherein a 2-cis-pentenoate is treated at from 20 to 250 °C in the presence of one or more compounds of the formula I

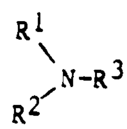

I

where $R^1$ is hydrogen or $R^1$ and $R^2$ are each cycloalkyl of 5 to 8 carbon atoms or alkyl of 1 to 6 carbon atoms, and $R^1$ and $R^2$ together with the nitrogen atom on which they are substituents may form a 5-membered to 7-membered ring which may additionally contain a further nitrogen or oxygen atom, and $R^3$ is hydrogen or a radical of the formula II

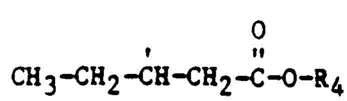

II

where $R^4$ is alkyl of 1 to 6 carbon atoms, cycloalkyl of 5 to 7 carbon atoms, aralkyl of 7 to 10 carbon atoms or aryl of 6 to 10 carbon atoms, with the proviso that a tertiary amine is present only when $R^3$ is a radical of the formula II and wherein from 0.05 to 0.2 mole of a compound of the formula I is used per mole of 2-cis-pentenoate.

**2.** A process as claimed in claim 1, wherein piperidine, pyrrolidine, morpholine or piperazine is used as the compound of the formula I and wherein from 0.05 to 0.2 mole of a compound of the formula I is used per mole of 2-cis-pentenoate.

3. A process as claimed in claim 1, wherein a 3-piperidino-, 3-pyrrolidino, 3-morpholino- or 3-piperazinovalerate is used as the compound of the formula I.

**Revendications**

1. Procédé d'isomérisation d'esters d'acide 2-cis-penténoïque en esters d'acide 2-trans-penténoïque, caractérisé en ce qu'on traite l'ester d'acide 2-cis-penténoïque à une température de 20 à 250 °C en présence d'au mains un composé de formule I

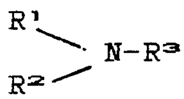

I

dans laquelle $R^1$ désigne un atome d'hydrogène ou, de même que $R^2$, un reste cycloalkyle à 5-8 atomes de carbone au un reste alkyle à 1-6 atomes de carbone, $R^1$ et $R^2$ pouvant former ensemble, avec l'atome d'azote qu'ils substituent, un noyau penta- à heptagonal qui peut contenir en plus un autre atome d'azote ou d'oxygène, et $R^3$ désigne un atome d'hydrogène ou un reste de formule II

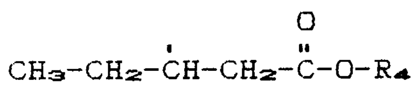

II

dans laquelle $R_4$ désigne un reste alkyle à 1-6 atomes de carbone, un reste cycloalkyle à 5-7 atomes de carbone, un reste aralkyle à 7-10 atomes de carbone, étant spécifié qu'une amine tertiaire n'est présente que quand $R^3$ désigne le reste de formule II, et en ce qu'on utilise, par mole d'ester d'acide 2-cis-penténoïque, de 0,05 à 0,2 mole de composés de formule I.

2. Procédé selon la revendication 1, caractérisé en ce qu'on utilise, comme composé de formule I, de la pipéridine, de la pyrrolidine, de la morpholine ou de la pipérazine.

3. Procédé selon la revendication 1, caractérisé en ce qu'on utilise, comme composé de formule I, un ester d'acide 3-pipéridino-, 3-pyrrolidino-, 3-morpholino- ou 3-pipérazinovalérique.